# EUROPEAN PATENT APPLICATION

(11) **EP 4 752 232 A1**
(43) Date of publication of application: **03.06.2026**
(21) Application number: 24216812.8
(22) Date of filing: 02.12.2024
(51) Int. Cl.: C12Q 1/6816

(54) **MODULAR CAPTURE CONSTRUCT AND METHOD FOR DETECTING A PLURALITY OF ANALYTES**

(71) Applicant: Leica Microsystems CMS GmbH, 35578 Wetzlar (DE)
(72) Inventor: Alsheimer, Soeren, 35578 Wetzlar (DE)
(74) Representative: Schaumburg und Partner Patentanwälte mbB

(57) **Abstract**

A modular capture construct (100, 900, 1000) for capturing a plurality of analytes (402, 504, 604, 706) of a biological sample (1300, 1408) is provided. The modular capture construct (100, 900, 1000) comprises a plurality of modules (102a, 102b, 102c), and at least a first dye (112) and a second dye (114) arranged on the capture construct (100, 900, 1000) to provide an orientation indication. The modular capture construct further comprises at least a first plurality of capture regions (108a, 108b, 108c, 108d, 108e, 108f), each capture region (108a, 108b, 108c, 108d, 108e, 108f) comprising at least one affinity capture reagent (400, 502, 602, 702, 704) configured to capture one of the analytes (402, 504, 604, 706), wherein each module (102a, 102b, 102c) comprises at least one of the capture regions (108a, 108b, 108c, 108d, 108e, 108f) of the first plurality of capture regions (108a, 108b, 108c, 108d, 108e, 108f). Each module (102a, 102b, 102c) comprises a nucleic acid backbone (201). Further, each of the modules (102a, 102b, 102c) is hybridised to at least another one of the modules (102a, 102b, 102c). In a further aspect, a method for detecting a plurality of analytes of a biological sample by means of the modular capture construct is provided.

## Description

### Technical field

The invention relates to a modular capture construct comprising a plurality of modules and a method for detecting a plurality of analytes of a biological sample by means of the modular capture construct.

### Background

The human proteome contains a significant portion of proteins that are secreted from cells and collectively referred to as the cells' secretome. There is considerable interest in terms of basic and translational research as well as diagnostic applications in obtaining secretion profiles, especially from single cells. A recent study by Uhlen et al. (Science Signaling, 26 Nov 2019: Vol. 12, Issue 609, DOI: 10.1126jscisignal.aaz0274) found that the majority of secreted proteins are actually retained intracellularly, i.e. are sorted to intracellular organelles instead of being released from the cell. It is thus of great importance to be able to differentiate between proteins that enter the secretory pathway, but remain intracellular, from the ones that are actually secreted from the cell into the extracellular space or blood stream.

Fluorescence-based assays are commonly used in life science research and diagnostic applications to detect the presence or absence of target analytes. Such target analytes may also be referred to as molecular markers. Molecular markers of interest may be of the group of proteins (proteome level), RNA and in particular mRNA (tran-scriptome level), DNA (genome level), metabolites (metabolome level), secreted molecules (secretome level), neurotransmitters, hormones and other small molecules of interest.

Citing from Tsai et al. 2020: "Fluorescent cell barcoding (FCB) is a multiplexing technique for high-throughput flow cytometry (FCM). Although powerful in minimizing staining variability, it remains a subjective FCM technique because of inter-operator variability and differences in data analysis" (Tsai et al. J Immunol Methods. 2020 Feb;477:112667. doi: 10.1016/j.jim.2019.112667.) In FCB up to three dyes are used in four different concentrations to label cells in different wells of a microplate for example by coupling the dyes to reactive amine-groups on the cell surface, which yields different intensities and color combinations. Both the subjectiveness of the technique and inter-operator variability of this method are inherently related to the fact it is based on encoding a part of the information in the hues of dyes, i.e. in intensity variations for example in light-green, green, dark-green, which severely limits the applicability of this technique.

In view of this it remains desirable to enable efficiently capturing a large number of molecular markers, in particular for single cells analysis. Further, techniques for the subsequent detection and analysis of these high number of molecular markers are not known, for example for secretome analysis.

### Summary

It is an object to provide a construct and method for capturing a plurality of analytes of a biological sample, which are efficient to generate and enable capturing of large numbers of analytes in a compact space.

The aforementioned object is achieved by the subject-matter of the independent claims. Advantageous embodiments are defined in the dependent claims and the following description.

In a first aspect, a modular capture construct for capturing a plurality of analytes of a biological sample is provided. The modular capture construct comprises a plurality of modules, and at least a first dye and a second dye arranged on the modular capture construct to provide an orientation indication. The modular capture construct further comprises at least a first plurality of capture regions, each capture region comprising at least one affinity capture reagent configured to (specifically) capture one of the analytes, wherein each module comprises at least one of the capture regions of the first plurality of capture regions. Each module comprises a nucleic acid backbone. Further, each of the modules is hybridised to at least another one of the modules.

The modular capture construct enables capturing the plurality of analytes at predetermined positions, the capture regions, on the nucleic acid backbones of the modules. Further, the modular capture construct enables capturing the analytes at a particularly high density. The capturing of the plurality of analytes enables efficient subsequent analysis of the plurality of analytes.

Thus, the modular capture construct may comprise several nucleic acid backbones, in particular, the modular capture construct may comprise as many nucleic acid backbones as it comprises modules.

Due to the modularity of the capture construct, the capture construct is efficiently generated, and the size of the capture construct can be easily adapted to the number of different analytes to be captured. In particular, each module may be individually assembled from nucleic acid strands and subsequently, the individual modules may be attached to each other to generate the modular capture construct. The plurality of modules may also be referred to as support modules or backbone modules. In particular, the modules and the modular capture construct provide a stable support, on which to capture the analytes.

The hybridisation of modules is based on intermolecular forces between the nucleic acid backbones, in particular of respective nucleotides, of adjacent modules. For example, the hybridisation is based on base-pairing between nucleotides of adjacent modules. Thus, the connection or hybridisation of modules is not based on or does not comprise covalent bonds.

The analytes may be a range of molecules. For example, an analyte may be a chemical species such as a metabolic product of the biological sample, or a cell signalling molecule of the biological sample. Further, the analyte may be a protein or peptide of the biological sample, such as a particular enzyme. Further, the analyte may be a hormone or a neurotransmitter. Further, the analyte may be a cell expressing a certain cell-surface protein or a specific combination of cell surface proteins. Further, the analyte may be a cell expressing a certain cell-surface protein or a specific combination of cell surface proteins with a certain glycosylation pattern. Further, the analyte may be a bacterium, an archaeon, a fungus (e.g. a yeast), or a virus. Further, the analyte may be a toxin or a heavy metal. Even further, the analyte may be a nucleic acid molecule, such as DNA or RNA, with a particular nucleic acid sequence. In particular, the analyte may be secreted by the biological sample, thus, enabling the capture of at least part of the secretome of the biological sample. Each affinity capture reagent may be configured to capture or bind to specifically one of the analytes. Each one of the first plurality of the capture regions may comprise at least one affinity capture reagent attached to the respective nucleic acid backbone in that capture region and configured to specifically bind to or capture one of the analytes. In other words, the capture construct may be configured such that each analyte is being captured in a particular one of the first capture regions. Therefore, each capture region, in particular a respective affinity capture reagent, may be configured to capture a different one of the analytes of the biological sample. The capture regions may also be called capture bands. The modular capture construct may also be called a modular nanoarray. Preferably, each of the modules comprises (at least) two capture regions of the first plurality of capture regions.

The biological sample may be a multicellular structure such as a cluster of live cells in particular a spheroid, or a single cell, enabling single cell analysis.

The first and second dyes may be arranged on specific modules to provide an orientation indication of the modular capture construct. The first dye may be arranged at or near an opposite end or side of the capture construct than the second dye. For example, the first dye may be attached to a first module and the second dye may be attached to a last module in a chain of modules. Thus, the first dye and the second dye may act as a first orientation indicator and as a second orientation indicator, respectively. The first and second dye enable determining the spatial orientation or the directionality of the capture construct, in particular of the modules. In this way, the orientation indicators enable spatial encoding. This means, different positions on the modules may be assigned to capture bands or capture regions that have reactivities to distinct analytes. For example, the first and second dyes each may be at least one fluorescent dye (of the same or of a predetermined different kind), attached to the nucleic acid backbone of the respective module. In particular, the first dye and the second dye are (optically) distinguishable from each other, for example based on their optical properties such as excitation wavelength, emission wavelength or emission lifetime. Thus, knowing the orientation of the modular capture construct from the first and second dye, the order of the plurality of modules and/or the first plurality of capture regions may be determined.

The nucleic acid backbone of each module provides a scaffold for the affinity capture reagents. Nucleic acid strands of the nucleic acid backbone of each module may be position-selectively functionalised. The positional resolution in this case is limited by the size of a nucleotide, which is in the range of a nanometre or below. Thus, the affinity capture reagents may be attached to the backbone at defined positions on the nucleic acid backbone of each module.

The nucleic acid backbone may comprise natural nucleic acids such as deoxyribonucleic acid, ribonucleic acid, for example. Additionally or alternatively, the nucleic acid backbone may comprise or essentially consists of nucleic acid analogues, such as locked nucleic acids.

Preferably, the nucleic acid backbone comprises or is a (single) main nucleic acid strand. This enables an efficient synthesis of the nucleic acid backbone of each module. In particular, the main nucleic acid strand is a continuous strand of covalently linked nucleotides. The main nucleic acid strand may alternatively be referred to as a scaffold strand.

Preferably, each module comprises at least one first nucleic acid strand comprising a first sequence complementary to a sequence of a corresponding first nucleic acid strand of another one of the modules. This enables hybridising of one module to another module of the plurality of modules. Each module may optionally comprise a plurality of first nucleic acid strands that each hybridise to a corresponding first nucleic acid strand of another one of the modules. This enables particularly robust connection between adjacent modules.

Preferably, some of the modules each comprise at least one second nucleic strand comprising a second sequence complementary to a sequence of a corresponding second nucleic acid strand of another one of the some of the modules. This enables generating the modular capture construct as a chain of chain of modules. For example, terminating modules of the chain of modules may only have the first nucleic acid strand described above or the second nucleic acid strand, and therefore only hybridise to one other module. Intermediary modules, which are situated along the chain of modules between two terminating modules, may have the first nucleic acid strand as well as the second nucleic acid strand. Thus, the intermediary modules may hybridise to two other modules and therefore link the two terminating modules as a chain of modules. The modules may optionally comprise a plurality of second nucleic acid strands that each hybridise to a corresponding second nucleic acid strand of another of the modules. This enables particularly robust connection between adjacent (intermediary) modules.

Preferably, the first dye and the second dye are each arranged on one of the terminating modules. This enables determining an orientation of the chain of modules of the modular capture construct, in particular determining an order of the capture regions on the modules.

In a particular embodiment, pairs of complementary sequences of the first nucleic acid strands and/or the second nucleic acid strands may be specific to pairs of modules. For example, each pair of adjacent modules of the chain of modules may have a unique complementary sequence of the first nucleic acid strands and/or the second nucleic acid strands. This enables a deterministic assembly of the modules, in particular of the order of the modules.

In another embodiment, the first nucleic acid strands and/or the second nucleic acid strands may be part of the nucleic acid backbone of one of the modules. In particular, the respective first sequences and/or second sequences may be part a sequence of the nucleic acid backbone, for example, of the main nucleic acid strand. In other words, the first nucleic acid strands and/or the second nucleic acid strands may be a single strand with the nucleic acid backbone of the respective module.

In an alternative embodiment, the first nucleic acid strands and/or the second nucleic acid strands may be separate nucleic acid strands, such as staple strand, that hybridise to the nucleic acid backbone of the respective module by complementary base-pairing.

Preferably, the first nucleic acid strand, in particularly the first sequence, is arranged towards or at a first end of the respective module, and/or wherein the second nucleic acid strand, in particularly the second sequence, is arranged towards a second end of the respective module. In particular, the first end and the second end of the modules are opposing ends of the respective modules. Thus, the first nucleic acid strands and/or the second nucleic acid strands may comprise further sequences that are complementary to respective parts of the nucleic acid backbone.

Preferably, each module comprises a plurality of staple (nucleic acid) strands configured to fold the nucleic acid backbone. In particular, the first nucleic acid strands and/or the second nucleic acid strands may be staple strands. This enables efficiently folding the nucleic acid backbone to generate a respective module.

In a particular embodiment, the nucleic acid backbone may comprise DNA origami. These DNA origami structures may range in size from a few nanometres into the micron range. For the fabrication of such DNA origami-based structures longer DNA molecules (scaffold strands) are folded at precisely identified positions by so called staple strands. The scaffold strand may be the main nucleic acid strand. The DNA origami may be designed to provide a self-assembly nucleic acid backbone of a particular predetermined shape. This enables an easy and reproducible synthesis and assembly of the nucleic acid backbone. For example, each module may be a separate DNA origami structure. Staple strands may be position-selectively functionalised, for example with affinity reagents. The positional resolution in this case is limited by the size of a nucleotide, which is in the range of a nanometre or below. This has been exploited in the prior art to generate fluorescent standards, wherein fluorescent dyes are connected to precisely located bands on the DNA origami. These standards are known as "nanoruler" and are used for the calibration of imaging systems like confocal or super resolution microscopes (e.g. STED), for example, as disclosed by US2014/0057805 A1.

Preferably, the DNA origami structure comprises at least one scaffold strand, such as the main nucleic acid strand, and multiple staple strands, wherein the staple strands are complementary to at least parts of the scaffold strand and configured to bring the scaffold strand into a predetermined conformation. In particular, the strands are oligonucleotides. This enables generating nucleic acid backbones with predetermined two- or three-dimensional shapes that can self-assemble. Further, this enables the site-specific placement of capture regions on the nucleic acid backbones. Preferably, the affinity capture reagents of the capture regions may be attached to staple strands of the nucleic acid backbones at predetermined positions. Staple strands allow the spatially precise functionalisation of the DNA origami at their respective locations on the DNA origami. Thus, each capture region may be located along the nucleic acid backbone of one of the modules at a particular staple strand or group of staple strands that are in close proximity. Since the staple strands are located at predetermined positions, the positions of the capture regions may equally be predetermined.

Preferably, the nucleic acid backbone, in particular, the main nucleic acid strand, of each module comprises between 1000 to 10000 nucleotides, more preferably, between 2000 to 7000 nucleotides. This enables efficiently generating the modules of the modular capture construct. In particular, this enables generating each module from a single nucleic acid strand, such as the main nucleic acid strand. Thus, each module may be generated from a nucleic acid strand with a number of nucleotides that can be synthesised in one piece.

Preferably, each module has a length, in particular a spatial extend in a particular direction, in a range between 30 to 250 nm. This enables a particularly compact capture construct.

Preferably, each module has an oblong, or rod-like shape. This enables a particularly compact capture construct. In particular, the length of each module may be along a longitudinal axis of the oblong shape.

Preferably, the modular capture construct comprises 2 to 10 modules, more preferably between 5 to 10 modules. Thus, the modular capture construct may be a chain of 2 to 10 modules, wherein each module is hybridised to at least one adjacent module. The choice of using a particular number of modules enables flexible adaptation of the modular capture construct to different applications, in particular adaptation to the number of analytes being captured.

Preferably, the affinity capture reagents are one of an antibody, an antibody fragment, an oligonucleotide, an aptamer, a peptide, a drug, and a toxin. This enables capturing a large variety of analytes with the affinity capture reagents. For example, the analyte may be a protein and the affinity capture reagent may be an antibody. In this case, the antibody may capture the protein by binding to a specific binding site or epitope of the protein. Generally, the affinity capture reagents of a particular capture region may bind to at least one particular binding site of the respective analyte.

Preferably, the modular capture construct further comprises a first plurality of affinity reporter reagents, each affinity reporter reagent comprising a first reporter tag and is configured to (specifically) bind to one of the analytes, wherein the first reporter tag is readable, to determine whether or not the respective analyte is captured by the respective affinity capture reagent. This enables determining the presence of a particular analyte by means of the modular capture construct. Since the modular capture construct comprises the first plurality of capture regions, a plurality of particular analytes may be captured and their presence determined. Similarly to the affinity capture reagents, each affinity reporter reagent may be configured to capture or bind to specifically one of the analytes. Therefore, each first capture region may comprise at least one affinity reporter reagent configured to specifically bind to one of the analytes associated with one of the first plurality of capture regions. Thus, for each target analyte one capture region with specific affinity capture reagents and specific affinity reporter reagents is provided.

Preferably, the affinity reporter reagents are selected from an antibody, an antibody fragment such as a single-domain antibody, an aptamer, a peptide, an oligonucleotide, an aptamer, a drug, and/or a toxin. This enables capturing and reporting a large variety of analytes with the modular capture construct. Generally, the affinity reporter reagents of a particular capture region may bind to at least one particular binding site of the respective analyte.

In a particularly preferred embodiment, the reporter tag, in particular the first reporter tag, is optically readable. For example, the reporter tag may comprise a fluorescent dye that is optically detectable. This enables determining by means of a microscope, in particular by imaging with a microscope, whether or not an analyte is captured by the respective affinity capture reagent and the respective affinity reporter reagent with its associated reporter tag is bound to that analyte.

Preferably, the reporter tag, in particular the first reporter tag, is an oligonucleotide and readable by sequencing. This enables determining, whether or not an analyte is captured by the respective affinity capture reagent and the respective affinity reporter reagent with its associated reporter tag is bound to that analyte.

Preferably, the affinity capture reagents are bound to the nucleic acid backbone, or the affinity capture reagents of a particular one of the capture regions comprise an oligonucleotide and the respective capture region comprises a complementary oligonucleotide to bind to the oligonucleotide of the affinity capture reagent.

In a preferred embodiment, the affinity capture reagents are covalently bound to the nucleic acid backbones of the modules, or the affinity capture reagents of a particular one of the capture regions comprise an oligonucleotide and the respective capture region comprises a complementary oligonucleotide to bind to the oligonucleotide of the affinity capture reagent. This enables particularly easy assembly of the modular capture construct, in particular of each of the modules of the capture construct.

The complementary oligonucleotide may be part of the nucleic acid backbones of the modules, preferably its staple strands. For example, each capture region of the capture construct may have complementary oligonucleotides only complementary to the oligonucleotides of the affinity capture reagent of the respective capture region. When adding the affinity capture reagents to the nucleic acid backbones, the affinity capture reagents then only bind to the corresponding complementary oligonucleotide of the respective capture region.

Examples of directly attaching or linking the affinity capture reagents to the staple strands, which enables a position-selective attachment to the nucleic acid backbone of the respective module, include direct chemical coupling through, for example, click chemistry reactions (e.g. Azide-Alkine) or through high-affinity interactions such as biotin-Streptavidin. In the latter case biotinylated staple strands and streptavidin-conjugated affinity capture reagents such as antibodies may be used.

Preferably, the modular capture construct comprises at least a second plurality of capture regions, each second capture region comprising at least one affinity capture reagent configured to (specifically) capture one of the analytes. This enables a particularly dense arrangement of capture regions on the capture construct. In particular, the second capture regions may capture analytes other than those captured by the first plurality of capture regions. Preferably, each module comprises at least one of the capture regions of the second plurality of capture regions.

Preferably, the modular capture construct further comprises a second plurality of affinity reporter reagents, each affinity reporter reagent comprising a second reporter tag and configured to (specifically) bind to one of the analytes, wherein the second reporter tag is readable, to determine whether or not the respective analyte is captured by the respective affinity capture reagent. This enables a particularly dense arrangement of capture regions on the capture construct. In particular, the second reporter tag is optically readable or readable by sequencing. Further, the second plurality of affinity reporter reagents may, in particular, bind to analytes, which are bound to one of the second plurality of capture regions.

Preferably, the modular capture construct extends, in particular essentially linearly, in one dimension and the first dye and the second dye are spaced apart from each other, or arranged on opposite ends of the modular capture construct. This enables determining the orientation of the modular capture construct. The dyes may be fluorescent dyes, for example. In particular, the first dye and the second dye have different properties, such as excitation wavelength, fluorescence emission wavelength, and/or fluorescence lifetime. For example, the first dye and the second dye may each be arranged on opposing terminating modules of a chain of modules of a modular capture construct.

Alternatively, the modular capture construct may preferably extend in two dimensions or three dimensions and the modular capture construct comprises at least a third dye, in particular a third orientation indicator. This enables determining the orientation of the modular capture construct.

Preferably, the first dye, the second dye and/or the third dye are (optically) distinguishable from the reporter tags, in particular in case the reporter tags are fluorescent reporter tags.

Preferably, the capture regions of the first plurality of capture regions are spaced apart from each other in a range from 1 nm to 2000 nm, more preferably in a range from 10 nm to 300 nm. This enables a particularly dense arrangement of capture regions on the nucleic acid backbones of the modules. In particular, the capture regions of each of the modules are spaced apart from each other in the range of 1 nm to 2000 nm.

Preferably, the capture regions of the first plurality of capture regions are spaced apart from the capture regions of the second plurality of capture regions in a range from 0.1 nm to 500 nm, more preferably in a range from 1 nm to 250 nm. This enables a particularly dense arrangement of capture regions on the nucleic acid backbones of the modules.

Preferably, the reporter tags comprise fluorophores, in particular with differing excitation wavelength, fluorescence emission wavelength, and/or fluorescence lifetime characteristics. In particular, the first reporter tags and the second reporter tags differ in their excitation wavelength, fluorescence emission wavelength, and/or fluorescence lifetime characteristics. This enables arranging capture regions particularly close together. In particular, this enables arranging a capture region of the first plurality of capture regions in a distance to a capture region of the second plurality of capture regions that is within the diffraction limit.

In a preferred embodiment, the affinity capture reagents of at least one of the capture regions are configured to bind one of the analytes at a single binding site of the analyte. This enables binding of the analyte with high sensitivity and specificity. For example, the analyte may be a protein and the affinity capture reagent may be an antibody. In this case, the antibody may capture the protein by binding to a specific binding site or epitope of the protein.

Preferably, at least one capture region comprises a first set of affinity capture reagents and a second set of affinity capture reagents, and wherein the first set of affinity capture reagent is configured to bind one of the analytes at a first binding site of the one analyte and the second set of affinity capture reagents is configured to bind the one of the analytes at a second binding site of the one analyte. This enables binding of the analyte with particularly high avidity. For example, a capture region may be configured such that an analyte may be bound at several different binding sites with the associated affinity capture reagents being specific to one of the binding sites and the capture region comprising affinity capture reagents for each of the binding sites. In an alternative example, an analyte may comprise several of the same binding site and the capture region may be configured such that the analyte may be bound by several of the associated affinity capture reagents specific to the one binding site.

In a particularly preferred embodiment, the first set of affinity capture reagents comprises first capture reagent dyes and the second set of affinity capture reagents comprises second capture reagent dyes. In particular the capture reagent dyes are fluorophores.

It is particularly preferred, that the first capture reagent dyes and the second capture reagent dyes are configured to be brought into proximity when the one analyte is captured by one of the affinity capture reagents of the first set of affinity capture reagents and by one of the affinity capture reagents of the second set of affinity capture reagents, and wherein the proximity enables an energy transfer between the respective capture reagent dyes. In particular, the proximity enables the formation of a FRET (fluorescence resonance energy transfer) pair or FRET n-tuples between the respective capture reagent dyes. This enables particularly high specificity when capturing and detecting analytes.

Preferably, the modular capture construct and the biological sample are embedded in or attached to a polymeric compound, in particular a hydrogel. This keeps the modular capture construct and the biological sample in close proximity and enables particularly easy handling of the biological sample with the modular capture construct.

In a further aspect, a method is provided for detecting a plurality of analytes of a biological sample, the method comprising the following steps: Incubation of the biological sample in the presence of at least one modular capture construct; Acquiring a readout of at least the one modular capture construct, in particular the capture regions of the at least one modular capture construct; Determining whether or not the respective analytes are captured by the respective affinity capture reagent.

Generally, a plurality of modular capture constructs may be incubated with or introduced into the biological sample, the modular capture constructs comprising capture regions being specific to respective analytes, in order to identify a large number of (different or the same) analytes at the same time. Preferably, an analyte is identified based on the reporter tags associated with the analyte in the readout. The modular capture constructs may be physically constituted before incubating them with the biological sample. Alternatively, individual elements, such as the modules and affinity capture reagents, of the modular capture constructs may be separately or sequentially incubated with or introduced into the biological sample thereby forming the modular capture construct when incubating the biological sample.

The method has the same advantages as the modular capture construct. Further, the method may be supplemented with the features of the modular capture construct described in this document, in particular, the features of the dependent claims of the capture construct.

### Terms

In the sense of this document the following terms are used in the following way:
**"Sample":** In the sense of this document "sample" may refer to a biological sample which may also be named a biological specimen including, for example blood, serum, plasma, tissue, bodily fluids (e.g. lymph, saliva, semen, interstitial fluid, cerebrospinal fluid), feces, solid biopsy, liquid biopsy, explants, model organisms (e.g. zebrafish, Drosophila, C. elegans), cells (e.g. prokaryotes, eukaryotes, archea), multicellular organisms (e.g. Volvox), suspension cell cultures, monolayer cell cultures, 3D cell cultures (e.g. spheroids, tumoroids, organoids derived from various organs such as intestine, brain, heart, liver, etc.), a lysate of any of the aforementioned, a virus. In the sense of this document "sample" may further refer to a volume surrounding a biological sample. For example, in assays, where secreted proteins such as growth factors, extracellular matrix constituents are being studied, the extracellular environment surrounding a cell up to a certain assay-dependent distance, may also be referred to as the "sample". Specifically, affinity reagents brought into this surrounding volume may be referred to as being "introduced into the sample".
**"Affinity reagent":** In the sense of this document the term "affinity reagent" and/or "affinity capture reagent" may in particular be an antibody, a single-domain antibody (also known as nanobody), a combination of at least two single-domain antibodies, an aptamer, an oligonucleotide, a morpholino, a PNA complementary to a predetermined RNA, DNA target sequence, a ligand (e.g. a drug or a drug-like molecule), or a toxin, e.g. Phalloidin a toxin that binds to an actin filament. In the sense of this document an affinity reagent may be configured to bind a target molecule or to an analyte with a certain affinity and specificity such that it can be said that the affinity reagent is substantially specific only to the target molecule or predetermined target structure.
**"Analyte":** In the sense of this document "analyte" or "predetermined target structure" may refer to a target molecule or a target structure or to an analyte, which may, for example, be a protein (e.g. a certain protein), an RNA sequence (e.g. the mRNA of a certain gene), a peptide (e.g. somatostatin), a DNA sequence (e.g. the a genetic locus or element), a metabolite (e.g. lactic acid), a hormone (e.g. estradiol), a neurotransmitter (e.g. dopamine), a vitamin (e.g. cobalamine), a micronutrient (e.g. biotin), a metal ion (e.g. metal and heavy metal ions like Cd(II), Co(II), Pb(II), Hg(II), U(VI)).
"Dye": In the sense of this document the terms "fluorescent dye", "fluorophore", "fluorochrome", "dye" are used interchangeably to denote a fluorescent chemical compound or structure and may be, in particular, one of the following: a fluorescent organic dye, a fluorescent quantum dot, a fluorescent dyad, a fluorescent carbon dot, a graphene quantum dot or another carbon-based fluorescent nanostructure, a fluorescent protein, a fluorescent DNA origami-based nanostructure. From the organic fluorescent dyes in particular derivatives of the following are meant by the term "fluorescent dye": xanthene (e.g. fluorescein, rhodamine, Oregon green, Texas), cyanine (e.g. cyanine, indocarbocyanine, oxacarbocyanine, thiacarbocyanine, merocyanine), squaraine rotaxane derivatives, naphthalene, coumarin, oxadiazole, anthracene (an-thraquinones, DRAQS, DRAQ7, CyTRAK Orange), pyrene (cascade blue), oxazine (Nile red, Nile blue, cresyl violet, oxazine 170), acridine (proflavine, acridine orange, acridine yellow), arylmethine (auramine, crystal violet, malachite green), tetrapyrrole (porphin, phthalocyanine, bilirubin), dipyrromethene (BODIPY, aza-BODIPY), a phosphorescent dye, or a luminescent dye. The following trademark groups designated commercially available fluorescent dyes, which may include dyes belonging to different chemical families CF dye (Biotium), DRAQ and CyTRAK probes (BioStatus), BODIPY (Invitrogen), EverFluor (Setareh Biotech), Alexa Fluor (Invitrogen), Bella Fluore (Setareh Biotech), DyLight Fluor (Thermo Scientific), Atto and Tracy (Sigma-Aldrich), FluoProbes (Interchim), Abberior Dyes (Abberior Dyes), Dy and MegaStokes Dyes (Dyomics), Sulfo Cy dyes (Cyandye), HiLyte Fluor (AnaSpec), Seta, SeTau and Square Dyes (SETA BioMedicals), Quasar and Cal Fluor dyes (Biosearch Technologies), SureLight Dyes (Columbia Biosciences), Vio Dyes (Milteny Biotec) [list modified from: https://en.wikipedia.org/wiki/Fluorophore]. From the group of fluorescent proteins in particular the members of the green fluorescent protein (GFP) family including GFP and GFP-like proteins (e.g DsRed, TagRFP) and their (monomerized) derivatives (e.g., EBFP, ECFP, EYFP, Cerulaen, mTurquoise2, YFP, EYFP, mCitrine, Venus, YPet, Super-folder GFP, mCherry, mPlum) are meant by the term "fluorescent dye". Further, from the group of fluorescent proteins the term "fluorescent dye" may include fluorescent proteins, whose absorbance or emission characteristics change upon binding of ligand like for example BFPms1 or in response to changes in the environment like for example redox-sensitive roGFP or pH-sensitive variants. Further, from the group of fluorescent proteins the term "fluorescent dye" may include derivative of cyanobacterial phycobiliprotein small ultra red fluorescent protein smURFP as well as fluorescent protein nanoparticles that can be derived from smURFP. An overview of fluorescent proteins can be found in Rodriguez et al. 2017 in Trends Biochem Sci. 2017 Feb; 42(2): 111-129 (https://www.ncbi.nlm.nih.gov/entrez/eutils/elink.fcgi?dbfrom=pub-med&retmode=ref&cmd=prlinks&id=27814948). The term "fluorescent dye" may further refer to a fluorescent quantum dot. The term "fluorescent dye" may further refer to fluorescent carbon dot, a fluorescent graphene quantum dot, a fluorescent carbon-based nanostructure as described in Yan et al. 2019 in Microchimica Acta (2019) 186: 583 and Iravani and Varma 2020 in Environ Chem Lett. 2020 Mar 10: 1-25 (https://www.ncbi.nlm.nih.gov/pmc/articles/PMC7088420). The term "fluorescent dye" may further refer to a fluorescent polymer dot (Pdot) or nanodiamond. The term "fluorescent dye" may further refer to a fluorescent dyad, like for example a dyad of a perylene antenna and a triangelium emitter as described in Kacenauskaite et al. 2021 J. Am. Chem. Soc. 2021, 143, 1377-1385. The term "fluorescent dye" may further refer to an organic dye, a dyad, a quantum dot, a polymer dot, a graphene dot, a carbon-based nanostructure, a DNA origami-based nanostructure, a nanoruler, a polymer bead with incorporated dyes, a fluorescent protein, an inorganic fluorescent dye, a SMILE, or a microcapsule filled with any of the aforementioned. The term "fluorescent dye" may further refer to a FRET-pair having at least one fluorescent dye as FRET donor and at least one fluorescent dyes as a FRET acceptor, or a FRET-triple, which is used to generate a three component Förster resonance energy transfer. In particular, the FRET-pair or FRET-triplet may be connected by a complementary linker or by a linker. The term "fluorescent dye" may further refer to a FRET n-tupel of physically connected dyes.
**"Readout device":** The term "readout device" may refer to a device used to perform fluorescence multicolour reading or imaging. A readout device typically includes at least one excitation light source, a detection system including at least one detection channel and may further contain filters and/or dispersive optical elements such as prisms and/or gratings to route excitation light to the sample and/or to route emission light from the sample onto to a detector or onto an appropriate area of the detector. The detection system may comprise several detection channels, may be a spectral detector detecting multiple bands of the spectrum in parallel, or a hyperspectral detector detecting a contiguous part of the spectrum. The detection system contains at least one detector, which may be a point-detector (e.g. a photomultiplier, an avalanche diode, a hybrid detector), an array-detector, a camera, hyperspectral camera. The detection system may record intensities per channel as is typically the case in cytometers or may be an imaging detection system that records images as in the case of plate readers or microscopes. A readout device with one detector channel, for example a camera or a photomultiplier, may generate readouts with multiple detection channels using, for example, different excitation and emission bands.
**"Oligonucleotide":** in the sense of this document may refer to DNA, RNA, peptide nucleic acid, morpholino or locked nucleic acid, glycol nucleic acid, threose nucleic acid, hexitol nucleic acid or another form of artificial nucleic acid.
**"Point spread function":** The term "point spread function" may be used to denote the main maximum of the point spread function and unless otherwise denoted the term refers to the effective point spread function (PSF) of the imaging system, which is generally elliptical, i.e. the lateral resolution is better than the axial resolution, but may approach an almost spherical shape as more views are acquired from preferably equidistant angles.

### Short Description of the Figures

Hereinafter, specific embodiments are described referring to the drawings, wherein:
- Fig. 1: is a schematic view of modular capture construct comprising three modules,
- Fig. 2: is a schematic view of different affinity reagents of capture regions of the modular capture construct,
- Fig. 3: is a schematic view of affinity reporter reagents with directly attached dyes,
- Fig. 4: is a schematic overview of the elements of the modular capture construct,
- Fig. 5: is a schematic view of a first embodiment of a capture region of the modular capture construct,
- Fig. 6: is a schematic view of a second embodiment of the capture region,
- Fig. 7: is a schematic view of a third embodiment of the capture region,
- Fig. 8: is a schematic view of a column of illumination and detection points spread functions and a column of corresponding effective point spread functions,
- Fig. 9: is a detailed schematic view of capture regions of a modular capture construct,
- Fig. 10: is a detailed schematic view of pluralities of capture regions of a modular capture construct,
- Fig. 11: schematically illustrates read out data from the capture constructs,
- Fig. 12: is a schematic view of the capture construct according to Figure 1 with a biological sample embedded in a hydrogel bead, and
- Fig. 13: is a schematic view of sample containers with biological samples.

### Detailed Description

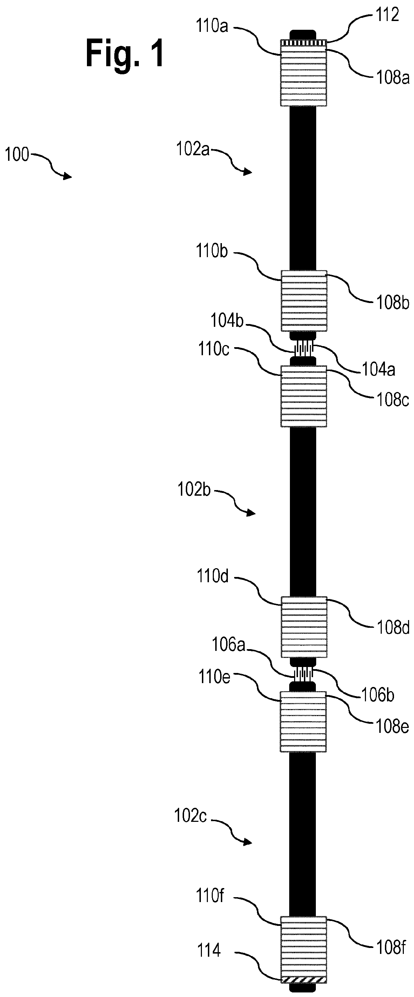

Figure 1 is a schematic view of a modular capture construct 100 comprising three modules 102a, 102b, 102c. The modules 102a, 102b, 102c are linked to each other in the form of a chain of modules. The terminating modules 102a, 102c are each linked to only one other intermediary module 102b, which is linked to the two terminating modules 102a, 102c. Thus, each of the modules 102a, 102b, 102c is linked to at least one other adjacent module 102a, 102b, 102c. Preferably, the modular capture construct 100 comprises between two to ten modules 102a, 102b, 102c. In particular, the modular capture construct 100 may comprise further intermediary modules 102b, for example up to seven additional intermediary modules 102b, which may each be linked to two other intermediary modules 102b.

Each module 102a, 102b, 102c comprises a nucleic acid backbone, which is linked to a nucleic acid backbone of an adjacent one of the modules 102a, 102b, 102c. The nucleic acid backbone of each of the modules 102a, 102b, 102c provides a scaffold for capture regions of the modular capture construct 100.

The nucleic acid backbones of adjacent modules 102a, 102b, 102c may be hybridised to each other based on complementary base-pairing. For example, the nucleic acid backbone of each module 102a, 102b, 102c may comprise at least one nucleic acid strand complementary to a respective at least one nucleic acid strand of an adjacent one of the modules 102a, 102b, 102c. In particular, the modules 102a, 102c may comprise several nucleic acid strands 104a, 106a, which comprise a sequence that is complementary to a sequence of several respective complementary nucleic acid strand 104b, 106b on the module 102b. Thus, when bringing the modules 102a, 102b, 102c together, the complementary nucleic acid strands 104a, 104b, 106a, 106b hybridise to each other and enable linking the modules 102a, 102b, 102c together to generate the modular capture construct.

In a particular embodiment, the (first) nucleic acid strands 104a, 104b may only be complementary to each other and the (second) nucleic acid strands 106a, 106b may only be complementary to each other. Thus, when bringing the modules 102a, 102b, 102c together, the modules 102a, 102b, 102c hybridise to each other in a predetermined or deterministic order.

In an alternative embodiment, the pairs of nucleic acid strands 104a, 104b and 106a, 106b may all be complementary to each other.

Each of the modules 102a, 102b, 102c preferably has an oblong, linear, or rod-like shape, as schematically shown in Fig. 1. The nucleic acid strands 104a, 104b, 106a, 106b are preferably arranged at ends of the modules 102a, 102b, 102c, in particular, at ends along a longitudinal axis of each of the modules 102a, 102b, 102c. In case of the intermediary module 102b, the nucleic acid strand 104b and the nucleic acid strand 106b are preferably arranged at opposite ends of the module 102b.

In a particular embodiment, the nucleic acid backbone of each module 102a, 102b, 102c may be a DNA origami. This enables generating the nucleic acid backbones in specific two- or three-dimensional shapes. In particular, the nucleic acid backbones may each comprise a main nucleic acid strand (scaffold strand), which is folded into precise two- or three-dimensional shapes using several (shorter) staple strands. To that end, the staple strands hybridise to specific sequences of the scaffold strand. Each staple strand may bind to two or more distant locations on the scaffold strand, causing the scaffold strand to fold into a desired shape. The specificity of Watson-Crick base pairing (A-T, C-G) drives the self-assembly process, with each staple strand pulling together distinct parts of the scaffold strand, creating crossovers and folds. The stiffness or flexibility of the DNA origami can be tuned. For example, areas with tightly packed helices and frequent crossovers are stiffer, while regions with sparse crossovers and fewer helices can introduce flexibility into the design.

Thus, the nucleic acid backbone of each module 102a, 102b, 102c is preferably a single DNA origami structure comprising a single main nucleic acid strand, which is folded into the rod-like shape by means of several staple strands.

In particular in case the nucleic acid backbones are DNA origami, the nucleic acid backbone of each of the modules 102a, 102b, 102c may have a number of nucleotides in a range between 1000 and 10000, preferably between 2000 and 7000.

The DNA origami structure, in particular within the size limits above, enables efficiently synthesising the individual modules 102a, 102b, 102c of the modular capture construct 100. Further, the provision of the nucleic acid strands 104a, 104b, 106a, 106b enables efficiently linking the individual modules 102a, 102b, 102c. Overall, this enables efficiently generating differently sized capture constructs comprising a varying number of modules 102a, 102b, 102c from a set of individual modules 102a, 102b, 102c. In particular, the above may contrast with a potential alternative approach, in which an entire capture construct may be based on only a single DNA origami, for example as described in the document PCT/EP2022/058640. Such an approach would necessarily limit the overall size of that capture construct, due to a limit of DNA origami sizes that can efficiently or economically be generated.

In a particular embodiment, the nucleic acid backbone of each module 102a, 102b, 102c may comprise a (single) main nucleic acid strand, which comprises at least one of the nucleic acid strands 104a, 104b, 106a, 106b, in particular their respective complementary sequence. Thus, for example the module 102b may comprise a main nucleic acid strand, which comprises the nucleic acid strands 104b, 106b in a single nucleic acid molecule.

In an alternative embodiment, the nucleic acid strands 104a, 104b, 106a, 106b of a particular one of the modules 102a, 102b, 102c may be staple strands of the nucleic acid backbone of the particular one of the modules 102a, 102b, 102c. In particular in this case, the staple strands may be of a DNA origami of the nucleic acid backbone.

The modular capture construct 100 further comprises several pluralities of capture regions. Exemplarily, capture regions of a first plurality of capture regions are indicated in Fig. 1 with the reference signs 108a, 108b, 108c, 108d, 108e, 108f. Capture regions of an optional second plurality of capture regions are exemplarily indicated in Fig. 1 with the reference signs 110a, 110b, 110c, 110d, 110e, 110f.

Each capture region 108a-108f, 110a-110f is configured to specifically capture an analyte of a biological sample. The capture regions 108a-108f, 110a-110f comprise respective affinity capture reagents with each capture region 108a to 108f comprising affinity capture reagents that specifically bind a particular analyte. Thus, the modular capture construct 100 comprises sixty capture regions 108a-108f, 110a-110f to capture sixty different analytes.

The spacing of the capture regions 108a-108f, 110a-110f and/or first and second dyes 112, 114 may be such that their distance to each other is in the range of 250 to 500 nm, for example. The spacing may be chosen depending on the resolving power of a readout device used to read out the capture regions 108a-108f, 110a-110f and first and second dyes 112, 114 and may be in the range of 1 nm to 5 nm, 10 nm to 25 nm, 50 nm to 100 nm, 100 nm to 250 nm, 250 nm to 500 nm, or 500 nm to 1000 nm. The preferable ranges correspond to the lateral resolution achievable with different microscopic modalities such as for example single molecule localization microscopy (1 nm to 25 nm), structured illumination and STED microscopy (50 nm to 100 nm), high NA (numerical aperture) light microscopy (around 200 nm), and low NA light microscopy (around 500 nm).

The modular capture construct 100 further comprises a first dye 112 and a second dye 114 for providing an orientation indication of the modular capture construct 100.

The first and second dyes 112, 114 may be fluorescent dyes or fluorophores, in particular fluorescent proteins, for example. The first dye 112 and the second dye 114 are arranged on opposing ends of the modular capture construct 100. For example, the first dye is 112 is arranged on the module 102a, which terminates the capture construct 100 at a first end, and the second dye 114 is arranged on the module 102c, which terminates the capture construct 100 at a second end, opposite the first end. In particular, the first dye 112 and the second dye 114 are distinguishable by their optical properties, such as their excitation wavelength, emission wavelength, or emission lifetime. This enables differentiating between the first and the second dyes 112, 114 in an optical readout of the capture construct 100, for example generated by means of a microscope, or an imaging cytometer. This further enables distinguishing the ends of the modular capture construct 100 from each other. Thus, the first and second dyes 112, 114 may be used to determine the orientation, directionality or polarity of the modular capture construct 100, in particular of the modules 102a, 102b, 102c.

Based on the directionality, the first and second dyes 112, 114 generate a relative coordinate system for the modular capture construct 100, relative to which each capture region 108a-108f, 110a-110f may be identified. In case of the linear modular capture construct 100, each capture region 108a-108f, 110a-110f is placed at a unique location along the modular capture construct 100. Each capture region 108a-108f, 110a-110f may be assigned an index n with n=1, 2, 3, ..., based on the unique location of the respective capture region 108a-108f, 110a-110f. In addition, the first and second dyes 112, 114 and their corresponding unique dye characteristics may be used to identify the particular modular capture construct 100 from a variety of capture construct with first and second dyes with different dye characteristics. Thus, a plurality of different capture construct may be used when analysing a biological sample, each capture construct distinguishable from the other capture construct by its unique combination of first dye and second dye.

In order to read out the capture regions 108a-108f, 110a-110f, generally, the first and second dyes 112, 114 are read out as well, for example by means of a microscope. This enables identifying the individual capture regions 108a-108f, 110a-110f in the readout based on the index n, as described above.

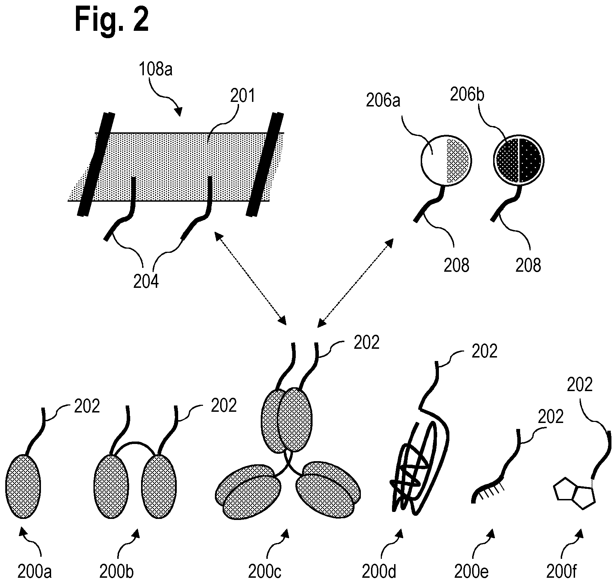

Figure 2 is a schematic view of different affinity reagents 200a to 200f. The affinity reagents 200a to 200f are, for example, single domain antibodies 200a, dimerised single domain antibodies 200b, antibodies 200c, aptamers 200d, oligonucleotide-based affinity reagents 200e, or small molecule-based affinity reagents 200f. These affinity reagents 200a to 200f may be used as the affinity capture reagents of one of the capture regions 108a-108f, 110a-110f. Here, the capture region 108a is exemplarily shown.

The affinity reagents 200a to 200f may be attached to a nucleic acid backbone 201 of the respective module 102a, 102b, 102c. Thus, an area of the nucleic acid backbone 201, to which affinity reagents 200a-200f with specificity to a particular analyte are attached to the nucleic acid backbone 201, is a capture region 108a-108f, 110a-110f.

In a particular embodiment, the affinity capture reagents may comprise oligonucleotide tags 202 in order to attach them to the backbone 201. Further the capture regions 108a-108f, 110a-110f may comprise corresponding complementary oligonucleotide tags 204, in particular, in case the backbone 201 is DNA origami-based. The oligonucleotide tags 204 may in that case be included in the backbone 201 when designing and constructing the DNA origami backbone 201 such that the tags 204 are accessible on the structure or protrude from the structure at the specific locations of the capture regions 108a-108f, 110a-110f. For example, the staple strands of the DNA origami may comprise the tags 204. Since the staple strands are at known predetermined locations of the DNA origami, the affinity capture reagents may be attached to these known predetermined locations to form the capture regions 108a-108f, 110a-110f. Complementary tags 202, 204 may be used to assemble the capture construct. For example, the backbone 201 may be constructed with unique tags 204 for each capture region 108a-108f, 110a-110f and the tags 204 chosen such that they correspond to the unique complementary tags 202 of the affinity capture reagents of each capture region 108a-108f, 110a-110f. Thus, the capture regions 108a-108f, 110a-110f are an area of the backbone 201, in which affinity capture reagents are bound to the backbone.

Alternatively, the affinity capture reagents may be covalently attached to the backbone 201.

Further, the affinity reagents 200a to 200f may be used to generate affinity reporter reagents. In particular, this may be achieved by attaching a dye 206a, 206b to the affinity reagent 200a to 200f by complementary oligonucleotide tags 202, 208, as described above. The dyes 206a, 206b may be fluorescent dyes, such as fluorescein or a fluorescent protein. In addition, the dyes 206a, 206b may have different characteristics such as fluorescent emission characteristics, excitation characteristics or lifetime characteristics. The dyes 206a, 206b comprise the oligonucleotide tag 208 which may be attached to the complementary oligonucleotide tags 202 of the affinity reagents 200a to 200b to provide a corresponding affinity reporter reagent.

The use of oligonucleotide tags 202, 204, 208 enables creating libraries of affinity reagents 200a to 200b that can be mixed and matched according to a user's requirements to result in required affinity capture reagents and affinity reporter reagents. This enables the flexible and cost-effective assembly of affinity capture reagents on the nucleic acid backbones, as well as the assembly of suitable dye-conjugated affinity reporter reagents.

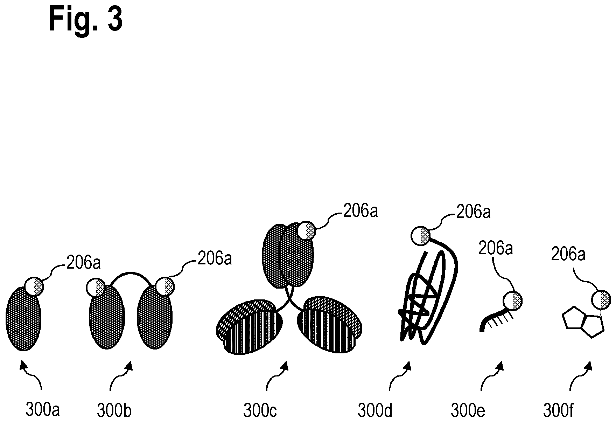

Figure 3 is a schematic view of affinity reporter reagents 300a to 300f with directly attached dyes 206a, 206b, which may be used alternatively to the affinity reporter reagents using oligonucleotide tags 202, 208. For example, the affinity reporter reagents 300a to 300f may have covalently attached dyes 206a, 206b.

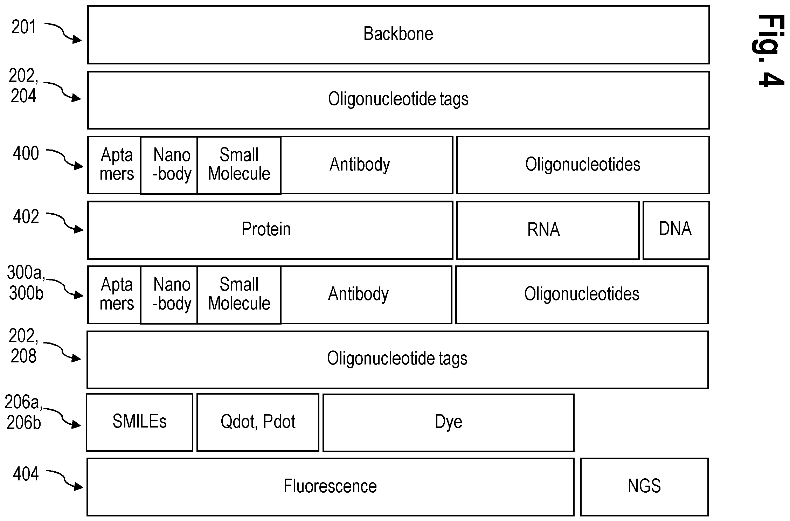

Figure 4 is a schematic overview of the elements of the modular capture construct 100, in particular of one of the capture regions 108a-108f, 110a-110f. Each capture region 108a-108f, 110a-110f has a plurality of affinity capture reagents 400 attached to the area of the nucleic acid backbone 201 of the respective module 102a, 102b, 102c. These affinity capture reagents 400 may be attached to the backbone 201 by linkers such as the oligonucleotide tags 202, 204. The affinity capture reagents 400 bind a respective analyte 402. In order to analyse the captured analytes 402, the affinity reporter reagents 300a to 300b may be attached to the analyte 402, the reporter reagents 300a to 300b comprising a dye 206a, 206b, which may be attached by linkers such as the oligonucleotide tags 202, 208. The linkers 202, 204, 208 are optional and may further be photocleavable or enzymatically cleavable, for example, with restriction enzymes, recombinases, endonucleases, CriSPR/CAS, Cre/loxP and similar. The readout 404 may be achieved by (next generation) sequencing (NGS) or fluorescence detection, in order to determine whether or not the analyte is bound to a particular one of the capture regions 108a-108f, 110a-110f. The readout may be achieved by sequencing when the reporter reagent 300a-300f comprises a sequen-cable oligonucleotide, for example. The individual elements shown in Figure 4 may be combined, for example, a particular analyte, such as a protein, may be captured by an antibody capture reagent and an antibody fragment reporter reagent may be used, with a fluorescent dye attached, to be read out by a microscope. Alternatively, the capture reagent may be a small molecule and the reporter reagent an antibody fragment.

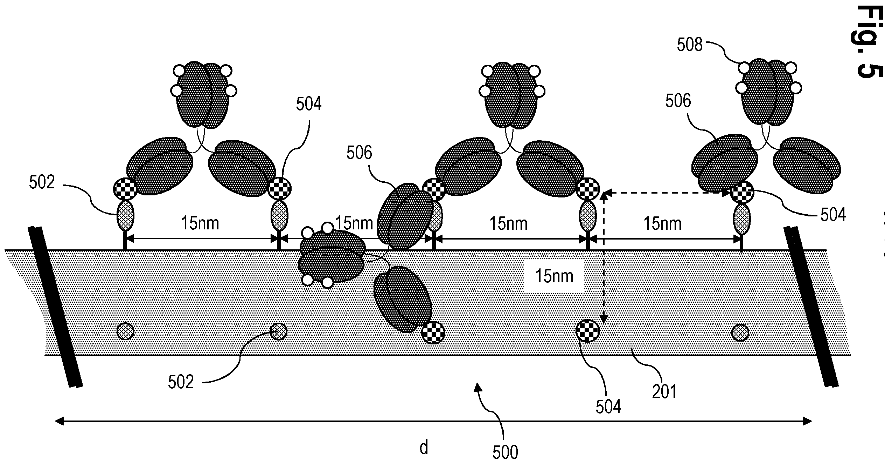

Figures 5 to 7 show specific examples of possible configurations of the modular capture construct 100, in particular of the capture regions 108a-108f, 110a-110f.

Figure 5 is a schematic view of a capture region 500. To the capture region 500 are attached multiple affinity capture reagents 502 in the form of single domain antibodies. The single domain antibodies specifically bind a particular analyte 504 of interest at a first binding site. Thus, the capture region 500 binds the analyte 504 where a capture reagent 502 is attached to the capture region 500.

In order to subsequently determine whether or not the analytes 504 are captured by the capture reagents 502, affinity reporter reagents 506 may be added. The affinity reporter reagents 506, in the form of antibodies, bind to the analyte at least at a second binding site. The affinity reporter reagents 506 comprise dyes 508, such as a fluorescent dye. Thus, the affinity reporter reagents 506 only accumulate at the capture region 500 when the analyte 504 is bound to the capture region 500. The presence of the affinity reporter reagent 506 and thus the analyte 504 may then be read out by the readout device as an optical signal of the dye 508. Only in the case that an optical signal of the dye 508 is detected in the capture region 500, it is determined that the analyte 504 is captured in the capture region 500.

More specifically, Figure 5 shows additional, optional features of the capture region 500. The use of a small affinity capture reagent 502, in the form of an antibody fragment, enables the spacing of analyte binding sites in a raster that has approximately 15 nm spacing, which corresponds roughly to the distance between the two binding sites, or paratopes, of conventional antibodies and is thus suited to create an avidity effect, which may increase the overall sensitivity of the assay substantially. Further, the three-dimensional arrangement of the affinity capture reagents 502 along and around the circumference of the rod-like backbone 201 increases the density of the binding sites of the affinity capture reagents 502 and consequently the affinity reporter reagents 506. This increases the signal to noise ratio of the optical signal when reading out the capture region 500. Finally, capturing a given analyte with two distinct affinity capture reagents and/or two distinct affinity reporter reagents, preferably each with different epitopes increases specificity of the assay and reduces sterical problems.

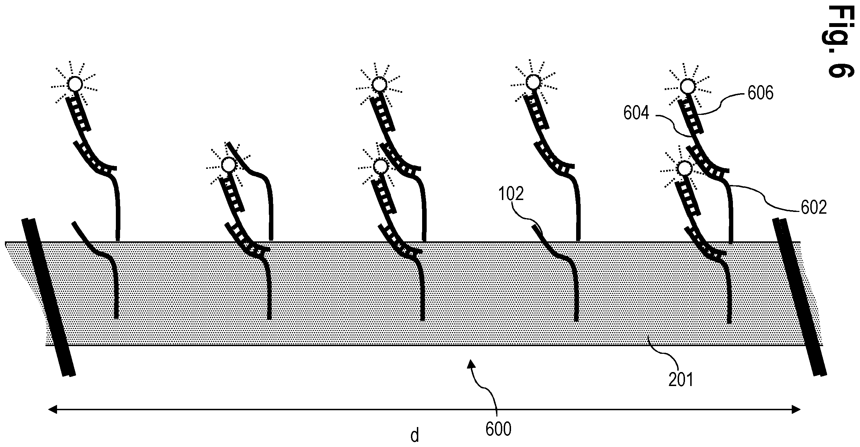

Figure 6 is a schematic view a capture region 600 with affinity capture reagents 602 in the form of oligonucleotides. The affinity capture reagent 602 is configured to bind an oligonucleotide analyte 604 comprising a complementary nucleic acid sequence. The analyte 604 bound to the capture reagent 602 may be determined by reading out the presence of an affinity reporter reagent 606 bound to the analyte 604 and comprising a complementary nucleic acid sequence to the analyte 604. The reagents 602, 606 and the analyte 604 may comprise DNA, RNA and/or LNA nucleotides. This enables detection of nucleic acid sequences with high sensitivity. This is particularly advantageous for numerous applications as nucleic acid sequences occur in bodily fluids or can be released from cells following lysis and potentially shearing of the DNA. This embodiment is also particularly advantageous for diagnostic testing in the context of liquid biopsies and their use to detect the presence of cancer. In this case circulating tumor DNA (ctDNA) target sequences may be detected. Further this embodiment is particularly advantageous for diagnostic testing of pathogen infection such as viral or bacterial infections including sepsis testing. Further areas of application are in pathogen detection in food and water quality testing and monitoring.

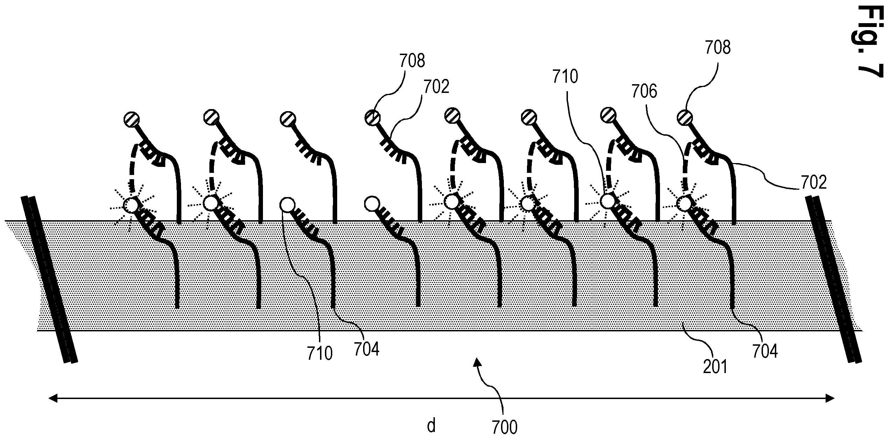

Figure 7 is a schematic view a capture region 700 with a first set of affinity capture reagents 702 in the form of oligonucleotides and a second set of affinity capture reagents 704 in the form of oligonucleotides. The affinity capture reagents 702, 704 are configured to bind an oligonucleotide analyte 706 at either a first complementary sequence or a second complementary sequence. Further the affinity capture reagents 702, 704 each have a corresponding first dye 708 or second dye 710 attached. When the analyte is bound to one of the affinity capture reagents 702 of the first set and one of the affinity capture reagents 704 of the second set, the first and second dyes 708, 710 of the respective affinity capture reagents 702, 704 are brought into close proximity. When the dyes 708, 710 are in close proximity they form a FRET-pair and a corresponding optical signal may be detected by the readout device. FRET refers to fluorescence resonance energy transfer. This increases the specificity of the detection of the analyte.

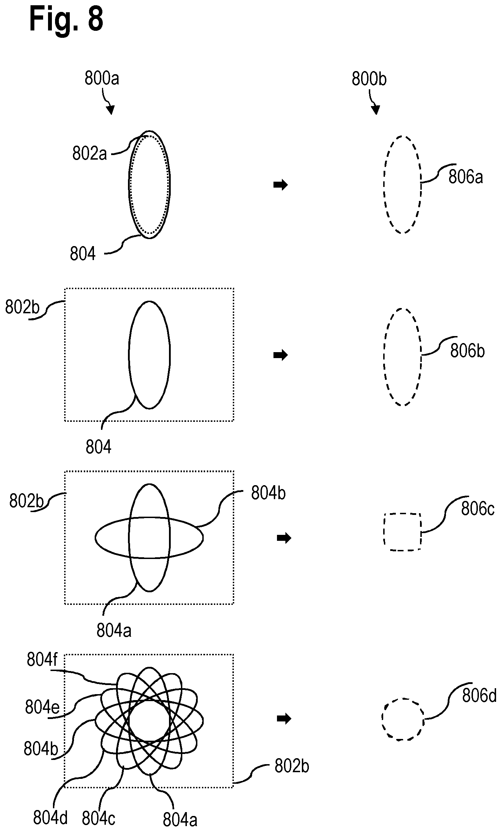

Figures 8 to 10 show options for reading out the capture construct 100, in particular the capture regions 108a-108f, 110a-110f, 500, 600, 700.

Figure 8 is a schematic view of a column of illumination and detection points spread functions (PSFs) 800a and a column of corresponding effective PSFs 800b. Unless noted otherwise PSF refers to the main maximum of the PSF in this document. Most microscopes illuminate and detect the sample through the same objective. In this case both the illumination PSF 802a and the detection PSF 804 are elliptical. In the case of light sheet fluorescence microscopy for example the illumination PSF 802b may be sheet-like and the detection PSF 804 may be elliptical, which still leads to an elliptical PSF provided that the detection PSF 804 is fully illuminated.

In the case of multi-view imaging with multiple detection PSFs placed at an angle 804a-804f, which may or may not be combined with light sheet illumination, effective PSFs 806c, 806d can be achieved, which are substantially improved over the elliptical PSFs 806a, 806b. Generally, an isotropic PSF improves the ability to resolve distinct capture regions 108a-108f, 110a-110f, 500, 600, 700 on a nanoarray and renders this also largely invariant to the orientation of the nanoarray. In other words, if an imaging system with an elliptical effective PSF 806a is used, then the resolving power in the axial direction (a) is lower than the in lateral direction (I). In the case of PSF 806d and PSF 806c the resolving power would be comparable in all room directions, which is not required for reading out nanoarrays, in particular capture regions 108a-108f, 110a-110f, 500, 600, 700, but may be preferable.

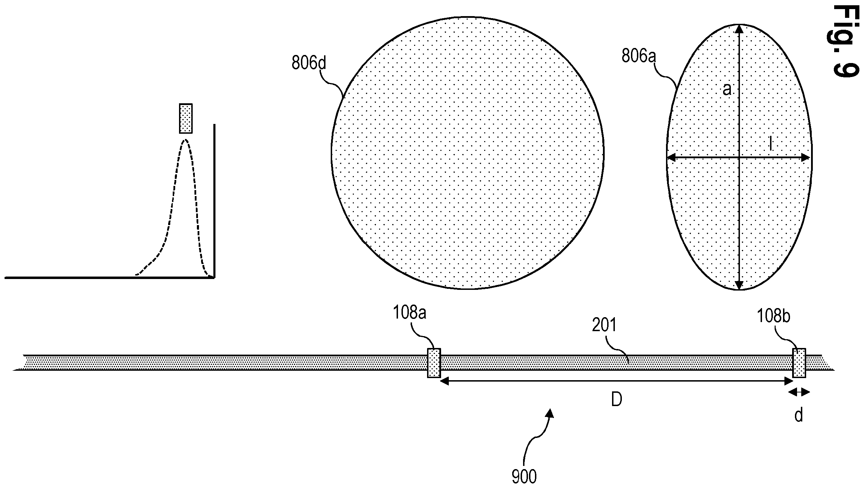

Figure 9 is a detailed schematic view of part of a module of a capture construct 900. The capture regions 108a, 108b are at distance (D) from each other of 500 nm, for example. The capture regions 108a, 108b may be read out by a readout device having the PSF 806a or the PSF 806d, as described for Figure 8. Importantly, the capture regions 108a, 108b are distanced from each other such that the readout device can resolve the capture regions 108a, 108b individually. Thus, all the affinity reporter reagents of the capture construct 900 may comprise the same dye.

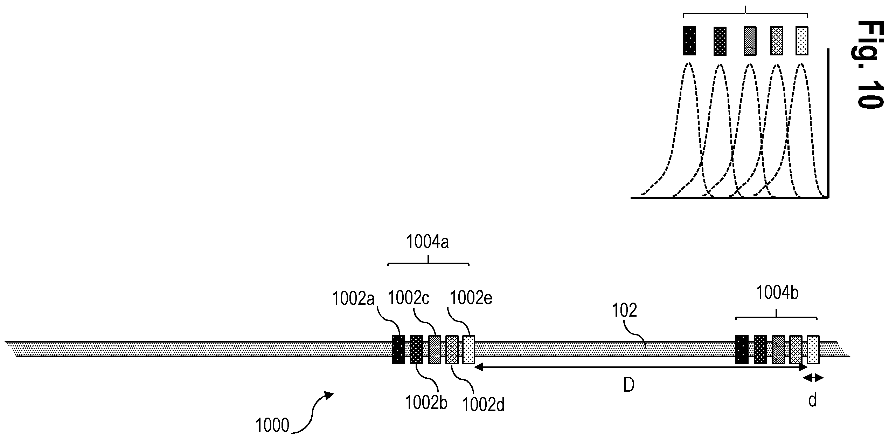

Figure 10 is a detailed schematic view of part of a module of a capture construct 1000 with several pluralities of capture regions. The capture construct comprises a first plurality 1002a, a second plurality 1002b, a third plurality 1002c, a fourth plurality 1002d and a fifth plurality 1002e of capture regions. The reference signs 1002a to 1002e refer to one of the capture regions of the respective plurality. The capture regions 1002a to 1002e are grouped with each group 1004a, 1004b comprising one of each of the capture regions 1002a to 1002e. The groups 1004a, 1004b are distance (D) from each other by 500 nm along the backbone 102. Each capture region 1002a to 1002e is approximately 25 nm wide (d) along the backbone 102.

The capture regions 1002a to 1002e may be read out by the readout device having the PSF 806a or the PSF 806d, as described for Figure 8 and 9. However, in order to differentiate between the capture regions 1002a to 1002e of each group 1004a, 1004b with the readout device, the affinity reporter reagents of the capture construct 1000 comprise different dyes. Specifically, the affinity reporter reagents of the plurality of capture regions 1002a to 1002e comprises a dye with characteristics unique to each of the plurality of capture regions 1002a to 1002e. This enables reading out individual capture regions 1002a to 1002e of a single group 1004a, 1004b.

This results in an increased density of capture regions 1002a to 1002e of capture construct 1000 and an accompanying vastly increased number of capture regions 1002a to 1002e compared to the capture construct 900 in Figure 9.

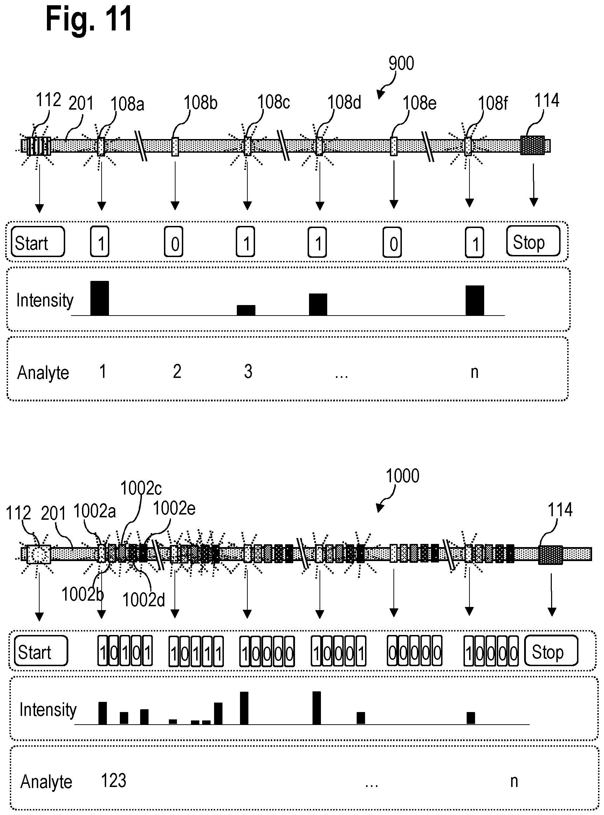

Figure 11 schematically illustrates read out data from the modular capture construct 900 and 1000. Each of the modular capture constructs 900, 1000 comprise four individual modules The optical signal determined from reading out the capture regions 108a to 108f, 1002a to 1002e may be categorised in a binary code of "0"s and "1"s, wherein a fluorescent signal from the analyte being present results in a "1" and no fluorescent signal when the analyte is absent results in a "0". A given sequence 0101010 for example can be interpreted or decoded for a given capture construct with known affinity reagents at each location of the capture regions and the directionality of the capture construct based on the first and second dyes 112, 114. This means, that the identities of analytes can be computed from a given sequence, or simply looked up in a memory file or database. In addition to providing a binary answer to the question whether a certain analyte was detected or not, the method provides intensity information, which can be used for relative quantification (i.e. analyte 1 has a 5x higher signal than analyte 2) or absolute quantification (i.e. the intensity read for analyte 1 corresponds to 10 dye molecules, which correspond to 5 analyte molecules for example).

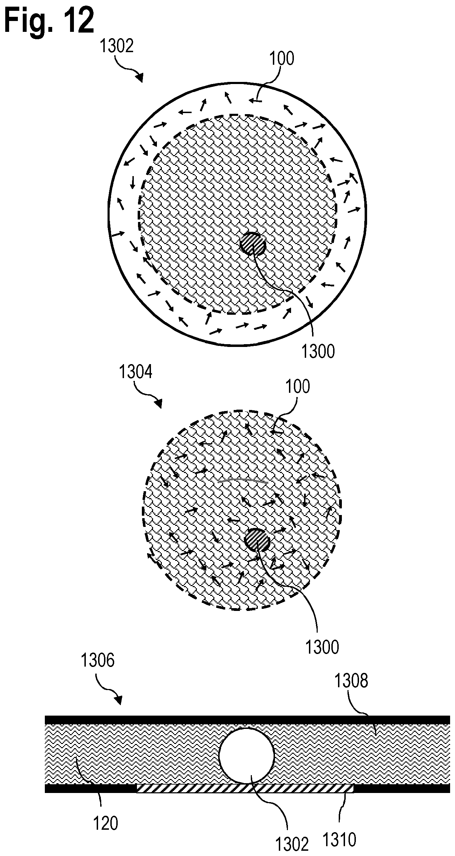

Figure 12 is a schematic view of the modular capture construct 100 with a biological sample 1300 embedded in a hydrogel bead 1302. The hydrogel bead 1302 is a multi-phasic hydrogel bead with a phase for cultivation of the biological sample 1300, such as a single cell or multiple cells in 3D cell culture, and at least one further layer which may be internal to or surrounding the cultivation phase/layer. The at least one further layer may be of the same or a different material. Alternatively, the biological sample 1300 and the capture construct 100 may be embedded in a single-phase hydrogel bead 1304

The biological sample 1300 encapsulated in the hydrogel bead 1302 may be cultivated and secrete proteins. For example, immune cells may be isolated following a liquid biopsy from a tumour patient or a patient that has an infectious disease, in order to study the immunophenotype, and/or immunorepertoire, and/or immunocompetence of the patient and to determine the best course of treatment. Secreted molecules are of great interest in this regard, for example as cytokines, and are indicative of the activation status of certain immune cells. The present invention leverages nanoarrays, which may be preferentially based on DNA-origami, carrying capture regions that capture analytes of interest.

The readout device may comprise a flow cell 1306 through which the hydrogel beads 1302, 1304 may flow in a liquid 1308. The flow cell 1306 comprises an optical window 1310 through which the hydrogel beads 1302, 1304, in particular the capture construct 100, 1000 may be imaged by an optical imaging device, such as a microscope.

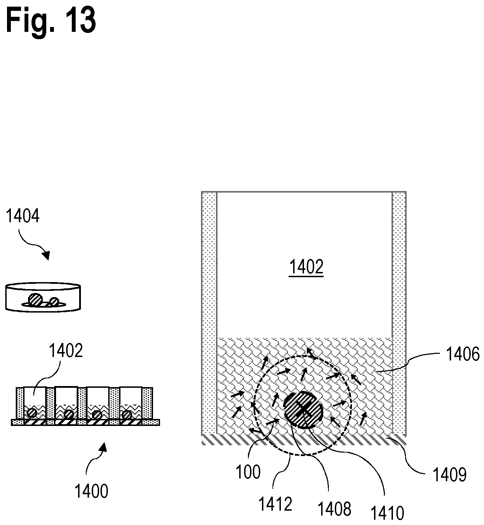

Figure 13 is a schematic view of sample containers with biological samples such as single cells embedded in a hydrogel gel. For example, a microplate 1400 with sample wells 1402 and a petri dish 1404. The capture construct 100 may be in suspension in a liquid or embedded in a hydrogel 1406 inside the sample well 1402 together with a biological sample 1408 such as a single cell or a multicellular structure under cultivation. The readout device may image the contents of the well 1402 through an optical window 1409 and determine the centre of mass 1410 of the biological sample 1408 and the capture constructs 100 that are situated within a predetermined radius 1412 of the centre of mass 1410 of the biological sample 1408.

Thus, in order to capture and detect secreted target analytes of the biological sample 1300, 1408 by means of the modular capture construct 100, the biological sample 1300, 1408 is incubated in the presence of the capture construct 100, or a plurality of the capture constructs 100, comprising capture regions with affinity capture reagents that bind the target analytes. This enables the analytes secreted by the biological sample 1300, 1408 to be captured by the capture construct 100, in particular the corresponding affinity capture reagents. Subsequently a readout of at least the capture construct 100, in particular the capture regions and the first and second dyes, is acquired. This readout may be acquired by means of an optical device, such as a microscope or an imaging flow cytometer. Based on the readout, the capture regions are then assessed to determine whether or not the respective target analytes are captured by the capture regions of the capture construct 100.

As used herein the term "and/or" includes any and all combinations of one or more of the associated listed items and may be abbreviated as "/".

Although some aspects have been described in the context of an apparatus, it is clear that these aspects also represent a description of the corresponding method, where a block or device corresponds to a method step or a feature of a method step. Analogously, aspects described in the context of a method step also represent a description of a corresponding block or item or feature of a corresponding apparatus.

### List of Reference Signs

100, 900, 1000 Modular capture construct
102a, 102b, 102c Module
104a First nucleic acid
104b Complementary first nucleic acid
106a Second nucleic acid
106b Complementary second nucleic acid
108a, 108b, 108c, 108d, 108e, 108f, 110a, 110b, 110c, 110d, 110e, 110f, 500, 600, 700, 1002a, 1002b, 1002c, 1002d, 1002e Capture region
112 First dye/orientation indicator
114 Second dye/orientation indicator
200a, 200b, 200c, 200d, 200e, 200f Affinity reagent
201 Nucleic acid backbone
202, 204, 208 Oligonucleotide tag
206a, 206b, 508, 708, 710 Dye
300a, 300b, 300c, 300d, 300e, 300f, 506, 606 Affinity reporter reagent
400, 502, 602, 702, 704 Affinity capture reagent
402, 504, 604, 706 Analyte
404 Readout
800a Column of illumination and detection points spread functions
800b Column of effective point spread functions
802a, 802b Illumination point spread function
804, 804a, 804b, 804c, 804d, 804e, 804f Detection point spread function
806a, 806b Elliptical effective point spread function
806c, 806d Isotropic point spread function
1004a, 1004b Group of capture regions
1300, 1408 Biological sample
1302, 1304 Hydrogel bead
1306 Flow cell
1308 Liquid
1310, 1409 Optical window
1400 Microplate
1402 Sample wells
1404 Petri dish
1406 Hydrogel or 3D cell culture matrix
1410 Centre of mass

## Claims

1. A modular capture construct (100, 900, 1000) for capturing a plurality of analytes (402, 504, 604, 706) of a biological sample (1300, 1408), comprising:
a plurality of modules (102a, 102b, 102c),
at least a first dye (112) and a second dye (114) arranged on the modular capture construct (100, 900, 1000) to provide an orientation indication, and
at least a first plurality of capture regions (108a, 108b, 108c, 108d, 108e, 108f), each capture region (108a, 108b, 108c, 108d, 108e, 108f) comprising at least one affinity capture reagent (400, 502, 602, 702, 704) configured to capture one of the analytes (402, 504, 604, 706), wherein each module (102a, 102b, 102c) comprises at least one of the capture regions (108a, 108b, 108c, 108d, 108e, 108f) of the first plurality of capture regions (108a, 108b, 108c, 108d, 108e, 108f),
wherein each module (102a, 102b, 102c) comprises a nucleic acid backbone (201), and wherein each of the modules (102a, 102b, 102c) is hybridised to at least another one of the modules (102a, 102b, 102c).

2. The modular capture construct according to claim 1, wherein the nucleic acid backbone (201) comprises a main nucleic acid strand.

3. The modular capture construct according to one of the preceding claims, wherein each module (102a, 102b, 102c) comprises at least one first nucleic acid strand (104a) comprising a first sequence complementary to a sequence of a corresponding first nucleic acid strand (104b) of another one of the modules (102a, 102b, 102c).

4. The modular capture construct according to claim 3, wherein some of the modules (102a, 102b, 102c) each comprise at least one second nucleic strand (106a) comprising a second sequence complementary to a sequence of a corresponding second nucleic acid strand (106b) of another one of the some of the modules (102a, 102b, 102c).

5. The modular capture construct according to one of the preceding claims 3 and 4, wherein the first nucleic acid strand (104a, 104b) is arranged towards a first end of the respective module (102a, 102b, 102c), and/or wherein the second nucleic acid strand (106a, 106b) is arranged towards a second end of the respective module (102a, 102b, 102c).

6. The modular capture construct according to one of the preceding claims, wherein the nucleic acid backbone (201) of each module comprises between 1000 to 10000 nucleotides.

7. The modular capture construct according to one of the preceding claims, wherein each module (102a, 102b, 102c) has a length in a range between 30 to 250 nm.

8. The modular capture construct according to one of the preceding claims comprising 2 to 10 modules (102a, 102b, 102c).

9. The modular capture construct according to one of the preceding claims, further comprising a first plurality of affinity reporter reagents (300a-300f, 506, 606), each affinity reporter reagent comprising a first reporter tag (206a, 206b, 508) and configured to bind to one of the analytes (402, 504, 604, 706), wherein the first reporter tag (206a, 206b, 508) is readable, to determine whether or not the respective analyte is captured by the respective affinity capture reagent (400, 502, 602, 702, 704).

10. The modular capture construct according to claim 9, wherein the reporter tag (206a, 206b, 508) comprises an oligonucleotide and readable by sequencing, and/or wherein the reporter tag (206a, 206b, 508) is optically readable.

11. The modular capture construct according to one of the preceding claims,
wherein the affinity capture reagents (400, 502, 602, 702, 704) are bound to the nucleic acid backbone (201), or
wherein the affinity capture reagents (400, 502, 602, 702, 704) of a particular one of the capture regions (108a, 108b, 108c, 108d, 108e, 108f) comprise an oligonucleotide and the respective capture region comprises a complementary oligonucleotide to bind to the oligonucleotide of the affinity capture reagent (400, 502, 602, 702, 704).

12. The modular capture construct according to one of the preceding claims comprising at least a second plurality of capture regions (110a, 110b, 110c, 110d, 110e, 110f), each second capture region comprising at least one affinity capture reagent (400, 502, 602, 702, 704) configured to capture one of the analytes (402, 504, 604, 706).

13. The modular capture construct according to one of the preceding claims, further comprising a second plurality of affinity reporter reagents (300a-300f, 506, 606), each affinity reporter reagent comprising a second reporter tag and configured to bind to one of the analytes (), wherein the second reporter tag is readable, to determine whether or not the respective analyte is captured by the respective affinity capture reagent (400, 502, 602, 702, 704).

14. The modular capture construct according to one of the preceding claims,
wherein the modular capture construct (100, 900, 1000) extends in one dimension and the first dye (112) and the second dye (114) are spaced apart from each other, or arranged on opposite ends of the modular capture construct (100, 900, 1000), or
wherein the modular capture construct (100, 900, 1000) extends in two dimensions or three dimensions and the modular capture construct (100, 900, 1000) comprises at least a third dye.

15. The modular capture construct according to one of the preceding claims, wherein the capture regions (108a, 108b, 108c, 108d, 108e, 108f) of the first plurality of capture regions are spaced apart from each other in a range from 1 nm to 2000 nm, preferably in a range from 10 nm to 300 nm.

16. The modular capture construct according to one of the preceding claims, wherein the capture regions (108a, 108b, 108c, 108d, 108e, 108f) of the first plurality of capture regions are spaced apart from the capture regions (110a, 110b, 110c, 110d, 110e, 110f) of the second plurality of capture regions in a range from 0.1 nm to 500 nm, preferably in a range from 1 nm to 250 nm.

17. A method for detecting a plurality of analytes of a biological sample, the method comprising the following steps:
incubation of the biological sample in the presence of at least one modular capture construct (100, 900, 1000) according to one of the preceding claims,
acquiring a read-out of at least the one modular capture construct (100, 900, 1000), in particular the capture regions (108a, 108b, 108c, 108d, 108e, 108f), and
determining whether or not the respective analytes (402, 504, 604, 706) are captured by the respective affinity capture reagent (400, 502, 602, 702, 704).
